# EUROPEAN PATENT APPLICATION

(11) **EP 3 305 166 A1**
(43) Date of publication of application: **11.04.2018**
(21) Application number: 16800097.4
(22) Date of filing: 26.05.2016
(51) Int. Cl.: A61B 1/00, A61B 1/04, A61B 17/94

(54) **MEDICAL MANIPULATOR SYSTEM**

(30) Priority: 27.05.2015 JP 2015107480
(71) Applicant: Olympus Corporation, Hachioji-shi Tokyo 192-8507 (JP)
(72) Inventor: ISODA, Takumi, Hachioji-shi Tokyo 192-8507 (JP)
(74) Representative: Gunzelmann, Rainer
(86) International application number: PCT/JP2016/065607
(87) International publication number: WO 2016/190393

(57) **Abstract**

The type of even an existing endoscope that does not hold property information is identified. Provided is a medical manipulator system (1) including: an endoscope (2) including an image-capturing unit; a manipulator (3) that holds the endoscope 2 at a distal end thereof and that moves the endoscope 2; an operating unit (4) for allowing an operator to input an operating command; a control unit (5) for controlling the manipulator (3) based on the operating command input to the operating unit (4); an insertion receiving member (6) having a through-hole (10) into which the endoscope (2) is inserted when the endoscope (2) is used; and a scope-type recognition unit for recognizing the type of the endoscope (2) based on an image, including an inner surface of the through-hole (10), that is acquired by the endoscope (2) in a state where the endoscope (2) is inserted into the through-hole (10).

## Description

### {Technical Field}

The present invention relates to a medical manipulator system.

### {Background Art}

There is a known endoscope apparatus in which, each time a different type of endoscope is mounted on the apparatus main body provided with an image capturing element and a light source, the apparatus main body reads out property information from a memory provided as a property information storing unit in the endoscope to store the property information of the endoscope and then sets the property information (refer to, for example, Patent Literature 1 below).

### {Citation List}

### {Patent Literature}

{PTL 1}
Japanese Unexamined Patent Application, Publication No. 2006-239204

### {Summary of Invention}

### {Technical Problem}

However, the endoscope apparatus in Patent Literature 1 is inconvenient in that when an existing endoscope not provided with a memory is mounted on the apparatus main body, the apparatus main body cannot read out the properties of the endoscope.

The present invention has been conceived in light of the above-described circumstances, and an object thereof is to provide a medical manipulator system that can identify the type of the endoscope even for an existing endoscope that does not store property information thereof.

### {Solution to Problem}

In order to achieve the above-described object, the present invention provides the following solutions.

One aspect of the present invention is a medical manipulator system including: an endoscope including an image-capturing unit; a manipulator that holds the endoscope at a distal end thereof and that moves the endoscope; an operating unit for allowing an operator to input an operating command; a control unit for controlling the manipulator based on the operating command input to the operating unit; an insertion receiving member that has a through-hole into which the endoscope is inserted when the endoscope is used; and a scope-type recognition unit for recognizing the type of the endoscope based on an image, including an inner surface of the through-hole, that is acquired by the endoscope in a state where the endoscope is inserted into the through-hole.

According to this aspect, when the endoscope is held at the distal end of the manipulator for use, by inserting the endoscope in an operating state into the through-hole of the insertion receiving member, the image including the inner surface of the through-hole is acquired by the image-capturing unit. Since the scope-type recognition unit recognizes the type of the endoscope based on the acquired image, the endoscope does not need to store the property information thereof, thus it is possible to identify the type, even when an existing endoscope is used.

Another aspect of the present invention is a medical manipulator system including: a manipulator that holds, at a distal end thereof, an endoscope including an image-capturing unit and that moves the endoscope; an operating unit for allowing an operator to input an operating command; a control unit for controlling the manipulator based on the operating command input to the operating unit; an insertion receiving member that has a through-hole into which the endoscope is inserted when the endoscope is used and that has an outer-surface-position detection unit for sequentially detecting a radial position of an outer surface of the endoscope inserted into the through-hole; and a scope-type recognition unit for recognizing the type of the endoscope based on a change over time in the radial position of the outer surface that is detected by the outer-surface-position detection unit when the endoscope is inserted into the through-hole.

According to this aspect, when using the endoscope held at the distal end of the manipulator, the radial position of the outer surface of the endoscope inserted into the through-hole is sequentially detected by the outer-surface-position detection unit provided on the insertion receiving member, as the endoscope in an operating state is inserted into the through-hole of the insertion receiving member. Since the scope-type recognition unit recognizes the type of the endoscope based on a change over time in the radial position of the outer surface of the endoscope being inserted into the through-hole, the endoscope does not need to store the property information thereof, thus it is possible to identify the type, even when an existing endoscope is used.

In the above-described aspect, the image may include an image of a mark on the inner surface of the through-hole, and the scope-type recognition unit may recognize the type of the endoscope based on the position of the mark in the image.

By doing so, the scope-type recognition unit can easily identify the type of the endoscope based on the position of the mark on the inner surface of the through-hole included in the image. That is, when a direct-viewing type endoscope is inserted into the through-hole, since the optical axis of the image-capturing unit is arranged in parallel to the axis of the through-hole, the acquired image shows the front opening of the through-hole at the center and the inner surface of the through-hole around the front opening.

On the other hand, when an oblique-viewing type endoscope is inserted into the through-hole, since the optical axis of the image-capturing unit is arranged to be oblique relative to the axis of the through-hole, the acquired image shows the front opening of the through-hole at a position shifted from the center of the image and the inner surface of the through-hole is shown around the front opening.

Therefore, by using the position of the mark on the inner surface of the through-hole including the opening, as an index, it is possible to easily recognize whether the type of the endoscope is a direct-viewing type or an oblique-viewing type with reference to.

In the above-described aspect, the mark may be an opening of the through-hole.

By doing so, whether the type of the endoscope is a direct-viewing type or an oblique-viewing type can be recognized easily based on the position of the opening of the through-hole, serving as a mark, without having to add a particular mark to the inner surface of the through-hole.

In the above-described aspect, the scope-type recognition unit may recognize the type of the endoscope based on a movement trajectory of the mark obtained by rotating the endoscope about a longitudinal axis thereof relative to the through-hole in a state where the endoscope is inserted into the through-hole.

By doing so, the type of the endoscope can be easily recognized based on the difference in movement trajectory of the mark obtained by rotating the endoscope about the longitudinal axis thereof, namely, the difference in the movement trajectory due to the difference in optical-axis direction between the direct-viewing type endoscope and the oblique-viewing type endoscope.

In the above-described aspect, the insertion receiving member may further include an insertion-amount detection unit for detecting the amount of insertion of the endoscope into the through-hole, and the scope-type recognition unit may recognize the type of the endoscope based on the amount of insertion detected by the insertion-amount detection unit and the radial position detected in relation to the amount of insertion.

By doing so, based on a change in the radial position detected by the outer-surface-position detection unit relative to the amount of insertion detected by the insertion-amount detection unit, it is possible to recognize the oblique angle of the distal end portion of the endoscope in a case where the endoscope is of an oblique-viewing type.

In the above-described aspect, the insertion receiving member may be a holding section that is attached to the distal end of the manipulator and that holds the endoscope.

In the above-described aspect, the insertion receiving member may be a trocar that penetrates through body surface tissue of a patient and that is mounted in the body surface tissue.

In the above-described aspect, based on the type of the endoscope recognized by the scope-type recognition unit, the control unit may switch control of the manipulator so that an optical axis of the image-capturing unit is moved in the same direction in response to an identical operating command input to the operating unit.

By doing so, it is possible to operate the manipulator in accordance with the type of the endoscope. That is, the direction in which the image acquired by the endoscope changes with respect to the moving direction of the endoscope is different, so that depending on whether the type of the endoscope is the direct-viewing type or the oblique-viewing type, by switching the control of the manipulator according to the type of the endoscope, it is possible to move the subject in the image in the same direction by an identical operation of the operating unit, regardless of the type of the endoscope. Therefore, the operator does not have to worry about the type of the endoscope, thereby enhancing the operability.

### {Advantageous Effects of Invention}

The present invention affords an advantage in that even for the existing endoscope that does not store property information, the type can be identified.

### {Brief Description of Drawings}

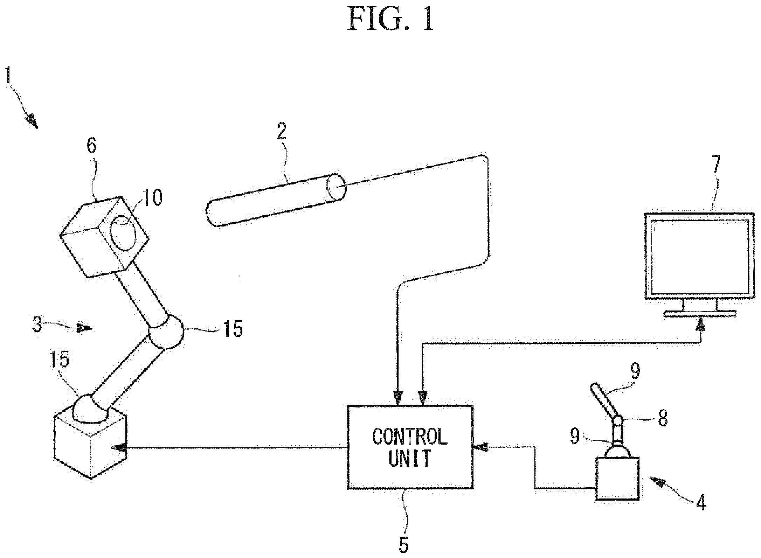

{Fig. 1}
   Fig. 1 is an overall configuration diagram showing a medical manipulator system according to one embodiment of the present invention.
{Fig. 2}
   Fig. 2 is a perspective view schematically showing a holder for mounting an endoscope probe on a manipulator of the medical manipulator system in Fig. 1.
{Fig. 3}
   Fig. 3 is a block diagram showing a control unit of the medical manipulator system in Fig. 1.
{Fig. 4A}
   Fig. 4A is a diagram showing an example image inside a through-hole of the holder, acquired by a direct-viewing-type endoscope probe of the medical manipulator system in Fig. 1.
{Fig. 4B}
   Fig. 4B is a diagram showing an example image inside the through-hole of the holder, acquired by an oblique-viewing-type endoscope probe (with a small oblique angle) of the medical manipulator system in Fig. 1.
{Fig. 4C}
   Fig. 4C is a diagram showing an example image inside the through-hole of the holder, acquired by an oblique-viewing-type endoscope probe (with a large oblique angle) of the medical manipulator system in Fig. 1.
{Fig. 5A)
   Fig. 5A is a diagram showing a manipulator on which an oblique-viewing-type endoscope probe is mounted in the medical manipulator system of Fig. 1.
{Fig. 5B}
   Fig. 5B is a diagram showing an example image acquired with an endoscope probe in the medical manipulator system of Fig. 1.
{Fig. 5C}
   Fig. 5C is a diagram showing the manipulator on which a direct-viewing-type endoscope probe is mounted in the medical manipulator system of Fig. 1.
{Fig. 6A}
   Fig. 6A is a diagram of a modification of Fig. 4A, showing an example image inside the through-hole of the holder, acquired by a direct-viewing-type endoscope probe.
{Fig. 6B}
   Fig. 6B is a diagram of a modification of Fig. 4B, showing an example image inside the through-hole of the holder, acquired by an oblique-viewing-type endoscope probe (with a small oblique angle).
{Fig. 6C}
   Fig. 6C is a diagram of a modification of Fig. 4C, showing an example image inside the through-hole of the holder, acquired by an oblique-viewing-type endoscope probe (with a large oblique angle).
{Fig. 7A}
   Fig. 7A is a diagram illustrating a difference in distance between the marks in the image of Fig. 6A when using a direct-viewing-type endoscope probe.
{Fig. 7B}
   Fig. 7B is a diagram illustrating a difference in distance between the marks in the image of Fig. 6B when using an oblique-viewing-type endoscope probe (with a small oblique angle).
{Fig. 7C}
   Fig. 7C is a diagram illustrating a difference in distance between the marks in the image of Fig. 6C when using an oblique-viewing-type endoscope probe (with a large oblique angle).
{Fig. 8}
   Fig. 8 is a perspective view showing a modification of the processing for recognizing the type of an endoscope probe.
{Fig. 9A}
   Fig. 9A is a diagram showing a movement trajectory of a mark in the through-hole of the holder, acquired by a direct-viewing-type endoscope probe, resulting from the processing in Fig. 8.
{Fig. 9B}
   Fig. 9B is a diagram showing a movement trajectory of the mark in the through-hole of the holder, acquired by an oblique-viewing-type endoscope probe (with a small oblique angle), resulting from the processing in Fig. 8.
{Fig. 9C}
   Fig. 9C is a diagram showing a movement trajectory of the mark in the through-hole of the holder, acquired by an oblique-viewing-type endoscope probe (with a large oblique angle), resulting from the processing in Fig. 8.
{Fig. 10}
   Fig. 10 is a longitudinal sectional view showing a trocar, as a modification of the insertion receiving member in Fig. 2.
{Fig. 11A}
   Fig. 11A is a diagram illustrating processing for inserting an oblique-viewing-type endoscope probe into the trocar in Fig. 10.
{Fig. 11B}
   Fig. 11B is a diagram showing an output waveform of a distance sensor acquired via the process of inserting the endoscope probe shown in Fig. 11A.
{Fig. 12A}
   Fig. 12A is a diagram illustrating the process of inserting a direct-viewing-type endoscope probe into the trocar in Fig. 10.
{Fig. 12B}
   Fig. 12B is a diagram showing an output waveform of the distance sensor, acquired via the process of inserting a direct-viewing-type endoscope probe shown in Fig. 12A.
{Fig. 13A}
   Fig. 13A is a diagram illustrating process of inserting an oblique-viewing-type endoscope probe into a modification of the trocar in Fig. 10.
{Fig. 13B}
   Fig. 13B is a diagram showing the relationship between the insertion amount of the endoscope probe and the output of the distance sensor acquired via the process of inserting the oblique-viewing-type endoscope probe shown in Fig. 13A.

### {Description of Embodiments}

A medical manipulator system 1 according to one embodiment of the present invention will now be described with reference to the drawings.

As shown in Fig. 1, the medical manipulator system 1 according to this embodiment includes: an endoscope 2; a manipulator 3 that holds this endoscope 2 at a distal end thereof and that moves the endoscope 2; an operating unit 4 for allowing an operator to input an operating command; a control unit 5 for controlling the manipulator 3 on the basis of the operating command input to the operating unit 4; a holder (insertion receiving member) 6 that is fixed to the distal end of the manipulator 3 and on which the endoscope 2 is mounted; and a monitor 7 for displaying the image acquired by the endoscope 2.

The endoscope 2 has an image-capturing unit (not shown in the figure) at the distal end thereof and is capable of acquiring an image of a subject disposed so as to face the distal end. The endoscope 2 is formed in an elongated shape. The types of the endoscope 2 include a direct-viewing type, in which a front image of an anterior direction in the longitudinal axis direction is acquired, and an oblique-viewing type, in which an image of an anterior oblique direction is acquired, and endoscopes with different oblique angles are available for the oblique-viewing type.

The manipulator 3 has, for example, a multi-joint structure and has a sufficiently high degree of freedom to set the position/orientation of the holder 6 fixed to the distal end thereof.

The operating unit 4 also has, for example, a multi-joint structure similar or substantially similar to that of the manipulator 3 and detects, as a motion command, the angle of each joint 9 for achieving the position/orientation of a handle 8 gripped and operated by the operator and outputs the motion command to the control unit 5.

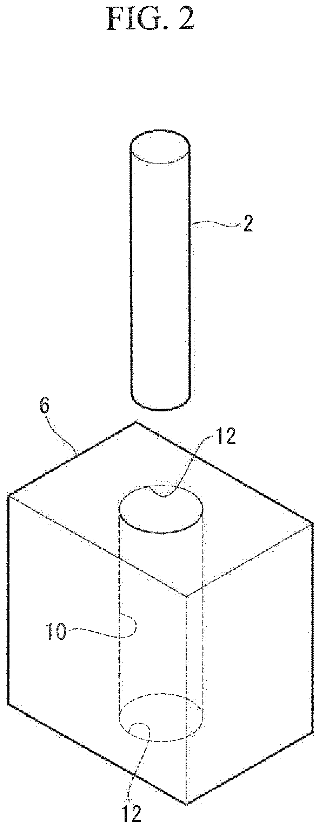

As shown in Fig. 2, the holder 6 includes a through-hole 10 into which the endoscope 2 is inserted to mount the endoscope 2 on the manipulator 3 and also a fixing mechanism, not shown in the figure, for fixing the endoscope 2 inserted into this through-hole 10. Furthermore, on the inner surface of the through-hole 10 of the holder 6, a mark for recognizing the type of the endoscope 2 with a scope-type recognition unit 11, to be described later, is provided. The mark may have any shape, and an opening 12 itself of the through-hole 10 formed in the holder 6 may be used as the mark.

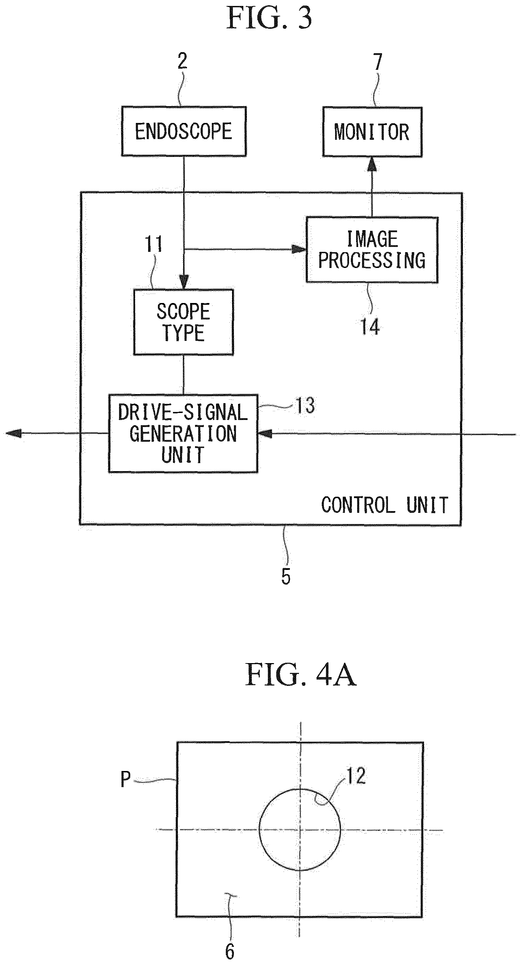

As shown in Fig. 3, the control unit 5 includes: a drive-signal generation unit (control unit) 13 that receives the motion command sent from the operating unit 4, generates a drive command signal for driving each joint 15 of the manipulator 3, and outputs the signal to the manipulator 3; the scope-type recognition unit 11 for recognizing the type of the endoscope 2 on the basis of an image that is acquired by the image-capturing unit when the endoscope 2 is inserted into the through-hole 10 of the holder 6; and an image processing unit 14 for processing the image acquired by the image-capturing unit and displaying the image on the monitor 7.

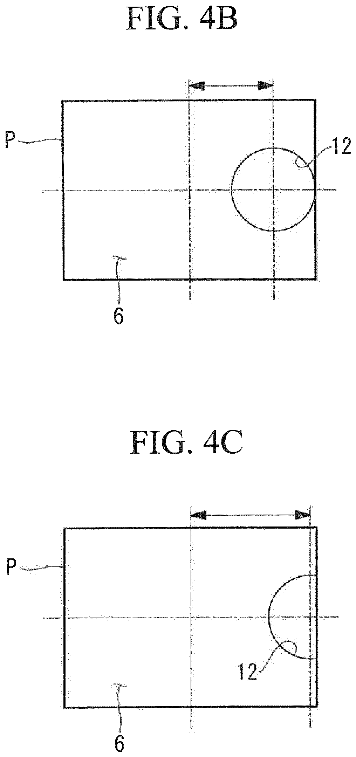

The scope-type recognition unit 11 recognizes the type of the endoscope 2 as the direct-viewing type when an image P that is acquired by the image-capturing unit when the endoscope 2 is inserted into the through-hole 10 of the holder 6 shows the opening 12 of the through-hole 10 at the center of the image P, as shown in Fig. 4A. The scope-type recognition unit 11 recognizes the type of the endoscope 2 as the oblique-viewing type when the image P that is acquired by the image-capturing unit when the endoscope 2 is inserted into the through-hole 10 of the holder 6 shows the opening 12 of the through-hole 10 at a position shifted in one direction relative to the center of the image P, as shown in Figs. 4B and 4C.

Furthermore, if the amount of shift of the opening 12 of the through-hole 10 relative to the center of the image P, as detected when the endoscope 2 is inserted into the through-hole 10 of the holder 6 by a prescribed depth, varies as shown in Fig. 4B and Fig. 4C, then the scope-type recognition unit 11 recognizes that the larger the amount of shift, the larger the oblique angle of the optical axis of the image-capturing unit relative to the longitudinal axis of the endoscope 2. For example, the oblique angle is 30° in the case of Fig. 4B, and the oblique angle is 45° in the case of Fig. 4C.

The drive-signal generation unit 13 receives information about the type of the endoscope 2 recognized by the scope-type recognition unit 11 and switches the motion of the manipulator 3.

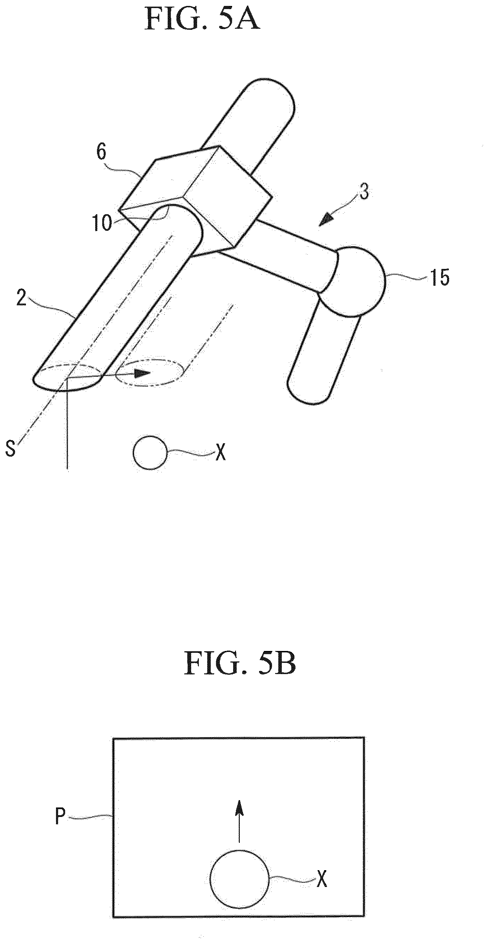

For example, if the endoscope 2 fixed to the holder 6 is of the oblique-viewing type, as shown in Fig. 5A, and the image P of a subject X as shown in Fig. 5B is acquired and displayed on the monitor 7, then it is necessary to move the distal end of the endoscope 2 in a direction orthogonal to an optical axis S in Fig. 5A, as indicated by the arrow, in order to move the subject X in a direction as indicated by the arrow on the image P.

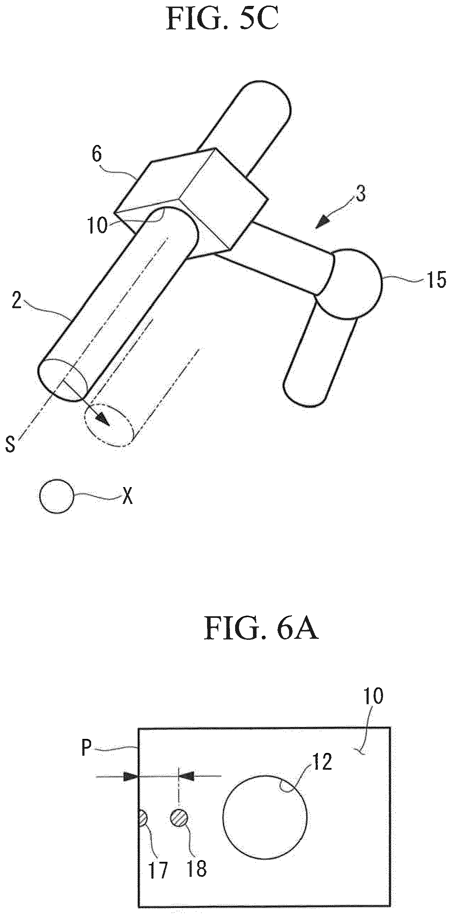

On the other hand, if the endoscope 2 fixed to the holder 6 is of the direct-viewing type, as shown in Fig. 5C, and the image P of the subject X as shown in Fig. 5B is acquired and displayed on the monitor 7, then it is also necessary to move the distal end of the endoscope 2 in a direction orthogonal to the optical axis S in Fig. 5C, as indicated by the arrow, in order to move the subject X in a direction indicated by the arrow on the image P. The movement direction in this case differs from that in the case of Fig. 5A, and the motion of the manipulator 3 for achieving this movement differs accordingly.

The drive-signal generation unit 13 is configured to control the manipulator 3, by switching the motion of the manipulator 3 according to the type of the endoscope 2, so that the subject X in the image P acquired by the endoscope 2 makes the same movement by an identical operation performed by the operator via the operating unit 4.

The operation of the medical manipulator system 1 according to this embodiment with the above-described configuration will be described below.

In order to observe the interior of the body of a patient using the medical manipulator system 1 according to this embodiment, first the power supply of the endoscope 2 is turned on, and then in a state where the image P can be acquired by the image-capturing unit provided at the distal end, the endoscope 2 is inserted by a prescribed amount from the distal end side into the through-hole 10 of the holder 6 provided at the distal end of the manipulator 3.

Then, the image P, including the inner surface of the through-hole 10, is acquired by the image-capturing unit, showing the opening 12 of the through-hole 10 of the holder 6 in the image P. As a result of this image P being sent to the scope-type recognition unit 11, the position of the opening 12 in the image P is calculated by image processing. If the center position of the opening 12 is disposed within a predetermined range from the center of the image as a result of image processing, then the type of the endoscope 2 is recognized as the direct-viewing type. Furthermore, if the center position of the opening 12 is shifted from the center of the image as a result of image processing, then the type of the endoscope 2 is recognized as the oblique-viewing type, and the oblique angle is recognized according to the amount of shift. Thereafter, the result of recognition in the scope-type recognition unit 11 is sent to the drive-signal generation unit 13.

In the drive-signal generation unit 13, the control of the manipulator 3 is switched on the basis of the sent information about the type of the endoscope 2.

Subsequently, the endoscope 2 inserted into the through-hole 10 of the holder 6 is fixed to the holder 6, and then the manipulator 3 is driven as a result of the operator operating the operating unit 4, thereby allowing the position of the subject X in the image P, which has been acquired by the endoscope 2 and displayed on the monitor 7, to be changed.

In this case, according to the medical manipulator system 1 of this embodiment, since control of the manipulator 3 is switched on the basis of information about the type of the endoscope 2, the operator, without having to care about the type of the mounted endoscope 2, can move the position of the image P displayed on the monitor 7 in the same direction by applying an identical operation to the operating unit 4.

In this manner, the medical manipulator system 1 according to this embodiment affords an advantage in that the operability can be enhanced because the operator does not need to change the operation applied to the operating unit 4 depending on the type of the endoscope 2. In addition, the operability can be enhanced in the same manner even with an existing endoscope 2 because the type of the endoscope 2 is recognized on the basis of the image P acquired by the endoscope 2, instead of making the endoscope 2 store identification information thereof.

This embodiment has been described by way of an example where control is switched according to the type of the endoscope 2. Alternatively, the recognized type of the endoscope 2 may be displayed to the operator so that the operator can change the operation to be performed according to the recognized type.

The opening 12 of the through-hole 10 of the holder 6 is used as the mark in the medical manipulator system 1 according to this embodiment. Alternatively, dot-shaped other marks 16 may be provided on the inner surface of the through-hole 10.

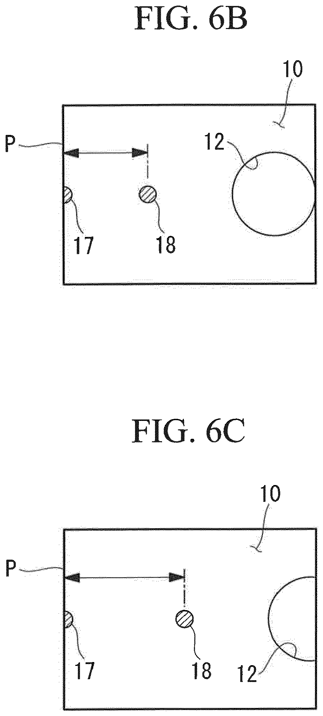

Further, as shown in Figs. 6A to 6C, two dot-shaped marks 17 and 18 spaced apart from each other in the longitudinal direction of the through-hole 10 may be provided, so that the type of the endoscope 2 can be recognized, for example, as the direct-viewing type, as the 30° oblique-viewing type, or as the 45° oblique-viewing type, according to the distance between the marks 17 and 18 as detected when the mark 17 on the near side is disposed at an extreme edge of the image.

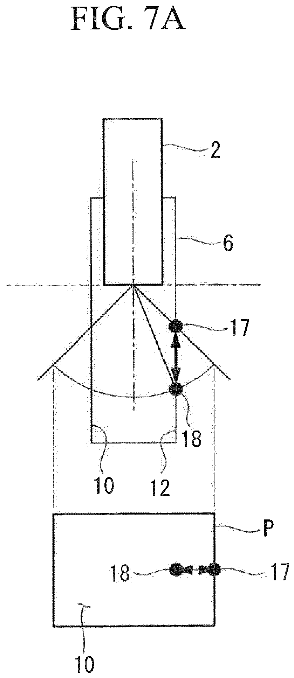

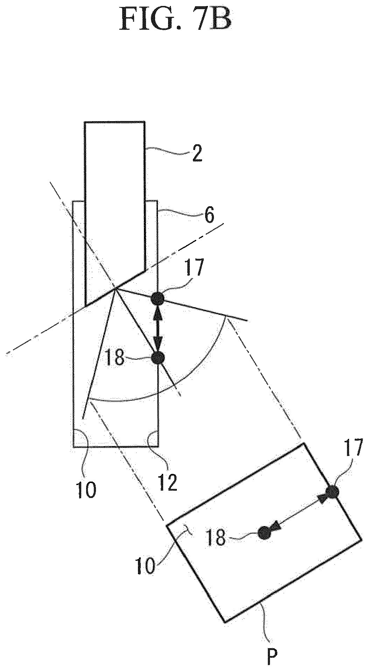

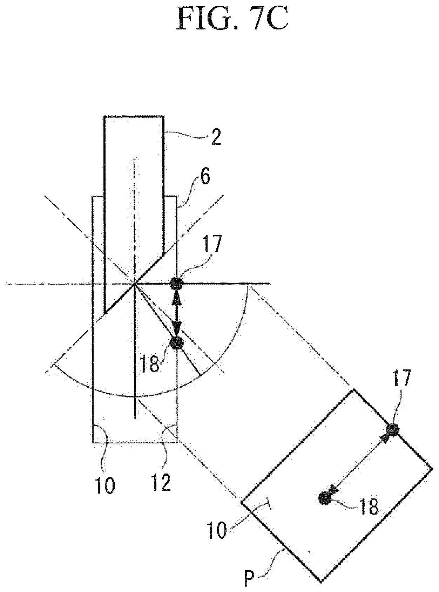

More specifically, as shown in Figs. 7A to 7C, the distance between the marks 17 and 18 is the smallest for the endoscope 2 of the direct-viewing type and increases in the order of the 30° oblique-viewing type and the 45° oblique-viewing type. Thus, the type of the endoscope 2 can be recognized easily by detecting the distance between the marks 17 and 18.

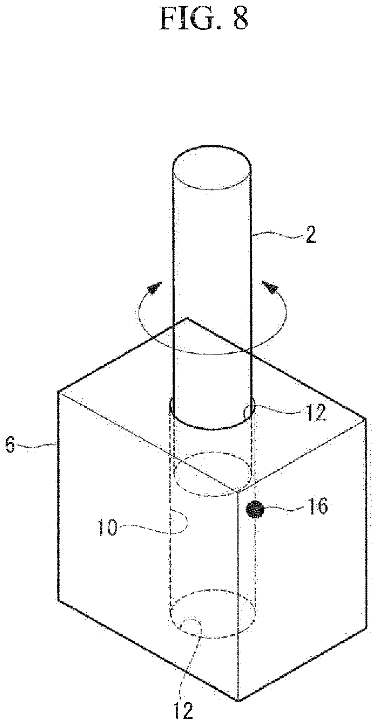

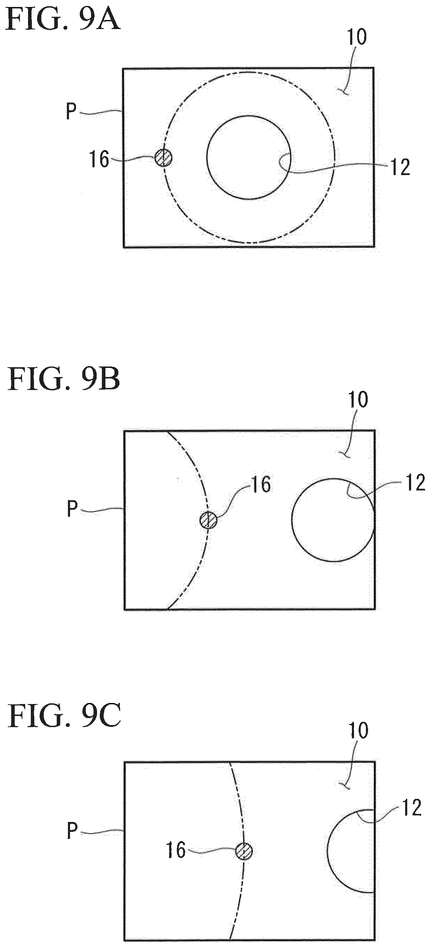

Furthermore, as shown in Fig. 8 and Figs. 9A to 9C, as a result of the endoscope 2 being rotated about the longitudinal axis of the endoscope 2 in a state where the endoscope 2 is inserted into the through-hole 10 of the holder 6, the dot-shaped mark 16 on the inner surface of the through-hole 10 moves with an arc-shaped movement trajectory. Therefore, detecting the radius of this movement trajectory also makes it possible to recognize the type of the endoscope 2 easily.

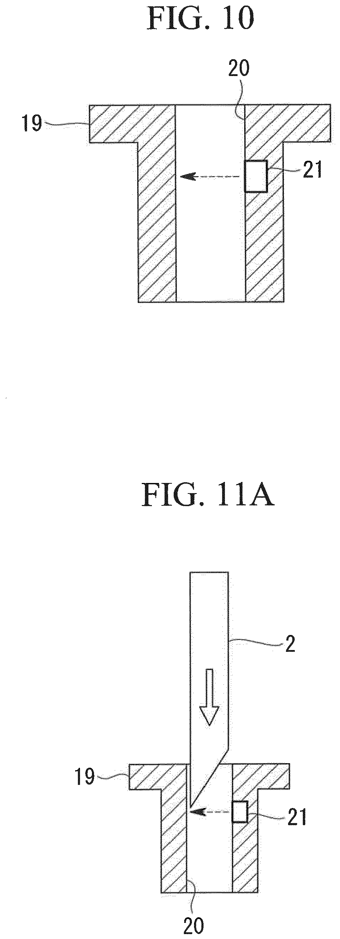

In this embodiment, the holder 6 provided with the through-hole 10 into which the endoscope 2 is inserted when the endoscope 2 is to be mounted on the manipulator 3 has been exemplified as the insertion receiving member. Alternatively, as shown in Fig. 10, a trocar 19 that is mounted in the body surface tissue of the patient in a state where the trocar 19 is installed so as to penetrate through the body surface tissue may be employed.

In the example shown in Fig. 10, the trocar 19 is provided with not only a through-hole 20 into which the endoscope 2 is inserted but also a distance sensor (outer-surface-position detection unit) 21, on the inner surface of the through-hole 20, that measures the distance from the inner surface to the outer surface of the inserted endoscope 2.

An example of the distance sensor 21 is an optical sensor that emits light and receives reflected light that is reflected at the outer surface of the endoscope 2.

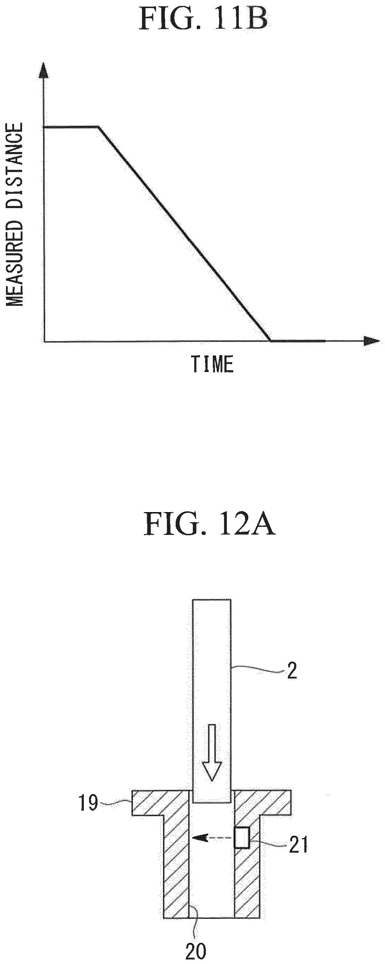

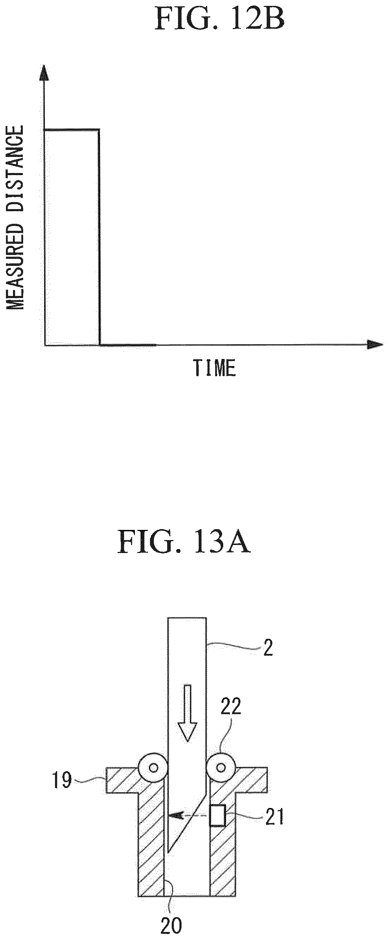

In this manner, since the distance from the inner surface of the through-hole 20 to the outer surface of the endoscope 2 is detected sequentially as the distal end of the endoscope 2 is inserted into the through-hole 20 of the trocar 19, the type of the endoscope 2 can be recognized as the oblique-viewing type if the distance changes gradually, as shown in Figs. 11A and 11B, or as the direct-viewing type if the distance changes abruptly, as shown in Figs. 12A and 12B.

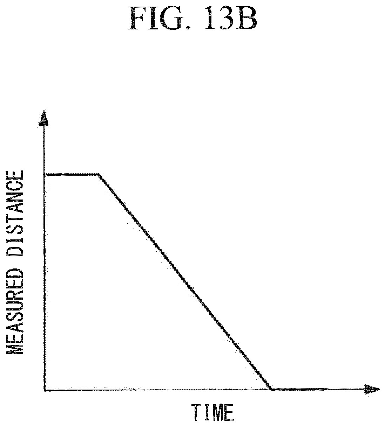

Furthermore, the trocar 19 may be provided with an encoder 22 for detecting the amount of insertion of the endoscope 2 as shown in Fig. 13A, so that the oblique angle of the oblique-viewing type can be determined on the basis of the relationship between the amount of insertion detected by the encoder (insertion-amount detection unit) 22 and the distance detected by the distance sensor 21. In short, as shown in Fig. 13B, the oblique angle of the endoscope 2 of the oblique-viewing type can be recognized directly from the slope of the graph representing distance vs. amount of insertion.

### {Reference Signs List}

- 1: Medical manipulator system
- 2: Endoscope
- 3: Manipulator
- 4: Operating unit
- 5: Control unit
- 6: Holder (insertion member, holding section)
- 10, 20: Through-hole
- 11: Scope-type recognition unit
- 12: Opening (mark)
- 16, 17, 18: Mark
- 19: Trocar (insertion member)
- 21: Distance sensor (outer-surface-position detection unit)
- 22: Encoder (insertion-amount detection unit)

## Claims

1. A medical manipulator system comprising:
an endoscope including an image-capturing unit;
a manipulator that holds the endoscope at a distal end thereof and that moves the endoscope;
an operating unit for allowing an operator to input an operating command;
a control unit for controlling the manipulator based on the operating command input to the operating unit;
an insertion receiving member that has a through-hole into which the endoscope is inserted when the endoscope is used; and
a scope-type recognition unit for recognizing the type of the endoscope based on an image, including an inner surface of the through-hole, that is acquired by the endoscope in a state where the endoscope is inserted into the through-hole.

2. A medical manipulator system comprising:
a manipulator that holds, at a distal end thereof, an endoscope including an image-capturing unit and that moves the endoscope;
an operating unit for allowing an operator to input an operating command;
a control unit for controlling the manipulator based on the operating command input to the operating unit;
an insertion receiving member that has a through-hole into which the endoscope is inserted when the endoscope is used and that has an outer-surface-position detection unit for sequentially detecting a radial position of an outer surface of the endoscope inserted into the through-hole; and
a scope-type recognition unit for recognizing the type of the endoscope based on a change over time in the radial position of the outer surface that is detected by the outer-surface-position detection unit when the endoscope is inserted into the through-hole.

3. The medical manipulator system according to claim 1, wherein the image includes an image of a mark on the inner surface of the through-hole, and
the scope-type recognition unit recognizes the type of the endoscope based on the position of the mark in the image.

4. The medical manipulator system according to claim 3, wherein the mark is an opening of the through-hole.

5. The medical manipulator system according to one of claims 3 and 4, wherein the scope-type recognition unit recognizes the type of the endoscope based on a movement trajectory of the mark obtained by rotating the endoscope about a longitudinal axis thereof relative to the through-hole in a state where the endoscope is inserted into the through-hole.

6. The medical manipulator system according to claim 2, wherein the insertion receiving member further includes an insertion-amount detection unit for detecting the amount of insertion of the endoscope into the through-hole, and
the scope-type recognition unit recognizes the type of the endoscope based on the amount of insertion detected by the insertion-amount detection unit and the radial position detected in relation to the amount of insertion.

7. The medical manipulator system according to one of claims 1 to 6, wherein the insertion receiving member is a holding section that is attached to the distal end of the manipulator and that holds the endoscope.

8. The medical manipulator system according to one of claims 1 to 6, wherein the insertion receiving member is a trocar that penetrates through body surface tissue of a patient and that is mounted in the body surface tissue.

9. The medical manipulator system according to one of claims 1 to 8, wherein, based on the type of the endoscope recognized by the scope-type recognition unit, the control unit switches control of the manipulator so that an optical axis of the image-capturing unit is moved in the same direction in response to an identical operating command input to the operating unit.
